# FASCICULE DE BREVET EUROPEEN

(11) **EP 2 934 339 B1**
(45) Date de publication et mention de la délivrance du brevet: **01.08.2018**
(21) Numéro de dépôt: 13830150.2
(22) Date de dépôt: 19.12.2013
(51) Int. Cl.: A61B 10/00, A61B 10/02

(54) **DISPOSITIF DE CONSERVATION D'UN ECHANTILLON BIOLOGIQUE**
VORRICHTUNG ZUR KONSERVIERUNG EINER BIOLOGISCHEN PROBE
DEVICE FOR PRESERVING A BIOLOGICAL SAMPLE

(30) Priorité: 20.12.2012 FR 1262398
(43) Date de publication de la demande: 28.10.2015
(73) Titulaire: Commissariat à l'Énergie Atomique et aux Énergies Alternatives, 75015 Paris (FR); Centre Hospitalier Universitaire de Grenoble Alpes, 38043 Grenoble Cedex 9 (FR); Université Grenoble Alpes, 38400 Saint Martin d'Hères (FR)
(72) Inventeur: COSNIER, Marie-Line, 38000 Grenoble (FR)
(74) Mandataire: Regimbeau
(86) Numéro de dépôt international: PCT/EP2013/077299
(87) Numéro de publication internationale: WO 2014/096138

(56) Documents cités:
- EP-A1- 2 325 619
- EP-A1- 2 333 511
- US-A- 6 085 907
- US-A1- 2005 011 790

## Description

### DOMAINE DE L'INVENTION

La présente invention concerne un dispositif de conservation d'un échantillon biologique.

### ARRIERE PLAN DE L'INVENTION

Le diagnostic par empreinte biologique, qui implique la mise en contact de la surface d'une zone de capture d'un dispositif de prélèvement avec un tissu à analyser, tend à se développer.

En effet, il est maintenant possible de réaliser des dispositifs de prélèvement de dimensions très faibles (typiquement, d'un diamètre inférieur ou égal à 1 mm), qui permettent de pratiquer des biopsies (prélèvements in-vivo) ou des prélèvements sur des tissus prélevés.

De tels dispositifs sont décrits notamment dans les documents WO 2006/082344 et WO 2008/0067871, auxquels on pourra se référer pour une description détaillée.

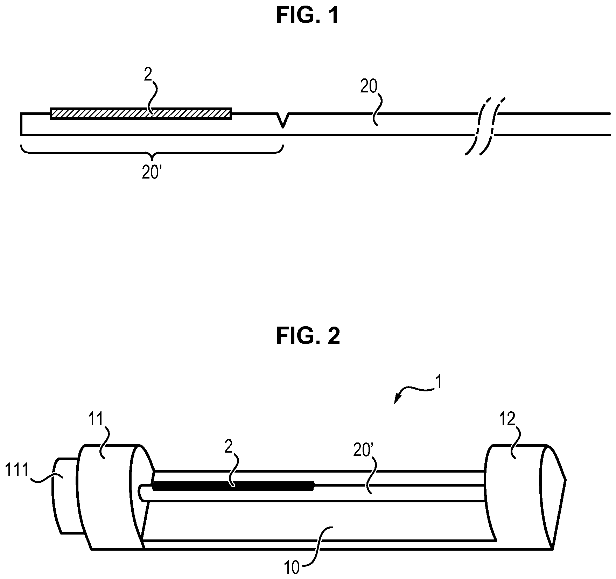

La figure 1 illustre un exemple d'un tel dispositif.

De préférence, le dispositif de prélèvement comprend une tige de manipulation, dont la longueur est choisie en fonction de l'utilisation visée et de la profondeur d'insertion nécessaire au prélèvement.

La tige 20 supporte à l'une de ses extrémités une zone de capture dont une surface est destinée à être mise au contact du tissu d'intérêt.

La surface de la zone de capture est avantageusement texturée et fonctionnalisée pour, d'une part, présenter une surface de contact vis-à-vis des cibles biologiques à prélever la plus grande possible, et d'autre part attirer et retenir lesdites cibles biologiques.

La zone de capture peut être constituée d'un élément distinct de la tige, qui est solidarisé de celle-ci par exemple par collage.

Elle peut également constituer une extrémité de la tige.

Généralement, la zone de capture peut être séparée de la tige.

L'élément 2 formant la zone de capture peut ainsi être une puce de silicium, bien qu'il ne soit pas limité à ce mode de réalisation particulier.

La partie 20' de la tige 20 portant l'élément de capture 2 peut être sécable, permettant de séparer l'élément de capture en vue de l'analyse ultérieure de l'échantillon.

La tige peut être insérée dans un guide (non illustré) qui définit la voie de passage du dispositif vers le site de prélèvement.

Après la réalisation d'un prélèvement avec un tel dispositif, l'échantillon biologique recueilli doit être traité rapidement afin d'éviter toute altération.

En effet, une fois que la tige a été retirée du guide, l'échantillon biologique se trouvant sur la surface de l'élément de capture est exposé à l'environnement du bloc opératoire ou du laboratoire où a eu lieu le prélèvement.

Or, l'échantillon ne peut pas nécessairement être analysé immédiatement et doit parfois être conservé un certain temps avant son analyse.

Les conditions de conservation de l'échantillon varient selon la nature de l'analyse qui doit être effectuée.

Ainsi, selon les cas, l'échantillon doit être conservé :
- à sec, à une température maîtrisée (par exemple -80°C ou 4°C),
- dans un liquide à température ambiante, ou à une température maîtrisée.

Pendant sa conservation, l'échantillon biologique, situé sur la zone de capture, doit également être protégé de toute contrainte mécanique, telle que frottement, torsion, etc. qui l'endommagerait.

Le document EP 2 333511 décrit un dispositif selon le préambule de la revendication 1.

Un but de l'invention est donc de concevoir un dispositif adapté pour la conservation et la protection d'un tel échantillon.

Ce dispositif doit être compatible avec tous les modes de conservation de l'échantillon mentionnés ci-dessus.

Dans le cas où l'échantillon doit être conservé dans un liquide, le dispositif doit être conçu pour minimiser le volume de liquide nécessaire.

Enfin, le dispositif devra permettre de visualiser la zone de capture (sur laquelle se trouve l'échantillon) de l'élément de capture.

### BREVE DESCRIPTION DE L'INVENTION

Conformément à l'invention, il est proposé un dispositif de conservation d'un échantillon biologique recueilli sur une surface d'un élément de capture agencé sur une portion détachable d'un dispositif de prélèvement biologique, comprenant :
- un corps,
- deux membres de maintien de ladite portion solidaires dudit corps, un premier membre étant pourvu d'un orifice pour le passage de l'élément de capture, lesdits membres de maintien étant agencés de sorte à maintenir entre eux ladite portion en évitant tout contact entre la surface de l'élément de capture et le corps. Le second membre de maintien est pourvu d'un orifice borgne adapté pour recevoir une extrémité de ladite portion.

Selon un mode de réalisation, lesdits orifices sont coaxiaux et le corps présente un méplat parallèle à l'axe desdits orifices.

De préférence, la distance entre lesdits membres de maintien est supérieure ou égale à la longueur de l'élément de capture et inférieure à la longueur de la portion.

Par ailleurs, le premier membre de maintien présente avantageusement, sur une face opposée au second membre de maintien, un élément de préhension adapté à la préhension du dispositif par un instrument.

Selon un mode de réalisation avantageux, le dispositif est inscrit dans un cylindre, l'élément de capture étant maintenu à l'intérieur dudit cylindre.

Le dispositif peut être réalisé en un matériau polymère, ledit matériau étant par exemple choisi parmi le polyoxyméthylène (POM), le polyétheréthercétone (PEEK), le polypropylène (PP) et le polyéthylène (PE).

Un autre objet de l'invention concerne un kit de prélèvement biologique comprenant :
- un dispositif de prélèvement biologique comprenant une tige et un élément de capture de cibles biologiques disposé sur une portion distale de la tige et détachable de ladite tige, et
- au moins un dispositif de conservation de l'échantillon biologique tel que décrit ci-dessus.

Un autre objet concerne un procédé de conservation d'un échantillon biologique prélevé au moyen d'un dispositif de prélèvement biologique comprenant une tige et un élément de capture de cibles biologiques disposé sur une portion distale de la tige et détachable de ladite tige.

Ledit procédé comprend les étapes suivantes :
- fourniture d'un dispositif de conservation de l'échantillon tel que décrit ci-dessus,
- introduction dudit dispositif dans un tube destiné à la conservation de l'échantillon,
- introduction de la portion distale dans l'orifice du premier membre de maintien et mise en place de ladite portion entre les membres de maintien,
- détachement de ladite portion de la tige,
- fermeture du tube par un bouchon.

Selon une forme d'exécution dudit procédé, on introduit un liquide dans le tube avant de le fermer par le bouchon.

De préférence, le diamètre extérieur du dispositif est sensiblement égal au diamètre intérieur du tube.

Enfin, la surface de l'élément de capture est avantageusement orientée à l'opposé du corps de sorte à être visible à travers la paroi du tube.

### BREVE DESCRIPTION DES DESSINS

D'autres caractéristiques et avantages de l'invention ressortiront de la description détaillée qui va suivre, en référence aux dessins annexés sur lesquels :
- la figure 1 illustre un exemple d'un dispositif de prélèvement biologique,
- la figure 2 est une vue en perspective d'un dispositif de conservation d'un échantillon biologique,
- la figure 3 présente des vues de côté, de dessus et de dessous du dispositif portant un élément de capture,
- la figure 4 est une vue schématique de la préhension du dispositif par une pince,
- la figure 5 est une vue schématique du dispositif introduit dans un tube.

### DESCRIPTION DETAILLEE DE L'INVENTION

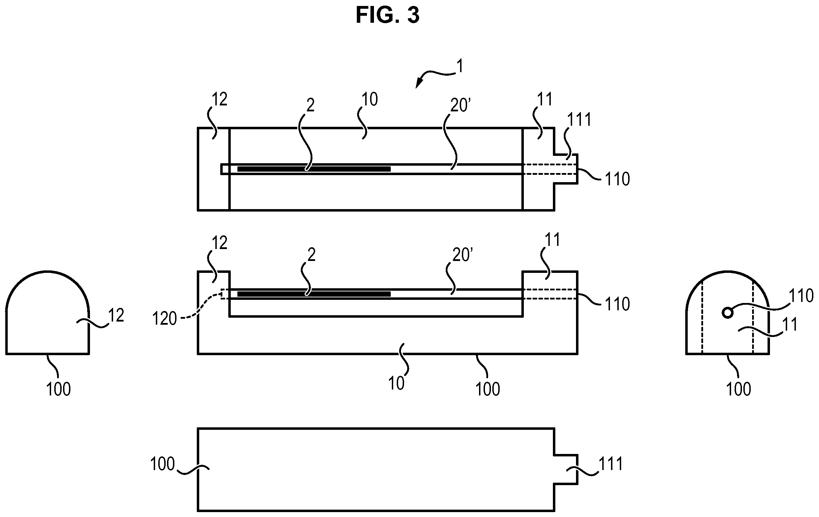

La figure 2 est une vue en perspective d'un dispositif de conservation 1 portant un élément de capture 2. La figure 3 présente des vues de dessus, de dessous et de côté dudit dispositif.

Comme mentionné plus haut, l'élément de capture 2 est généralement solidaire d'une portion 20' d'un dispositif de prélèvement biologique.

Par exemple, la portion 20' est une portion de distale d'une tige, qui peut être détachée du dispositif de prélèvement après la capture des cibles biologiques dans la région d'intérêt.

L'élément de capture et la portion 20' présentent une forme allongée, c'est-à-dire que leur longueur (dans la direction d'insertion du dispositif de prélèvement biologique) est bien supérieure à leur largeur et à leur épaisseur.

Le dispositif de conservation 1 est destiné à être inséré dans un tube obturable, assurant la protection de l'échantillon vis-à-vis de l'environnement extérieur et pouvant éventuellement être rempli d'un liquide.

Le dispositif 1 comprend d'une manière générale un corps 10 présentant des moyens de maintien de l'élément à distance du corps, de sorte à éviter tout contact entre la surface de capture et le corps, ainsi qu'avec le tube une fois que le dispositif 1 y a été introduit.

Les moyens de maintien se présentent sous la forme de deux membres 11, 12 solidaires du corps 10.

Dans le mode de réalisation illustré ici, les deux membres 11, 12 sont parallèles et écartés d'une distance supérieure à celle de l'élément de capture 2 tout en étant inférieure à la longueur de la portion 20', de sorte à recevoir et maintenir entre eux la portion 20' et l'élément de capture 2.

Le corps 10 remplit essentiellement une fonction de liaison mécanique rigide entre les deux membres 11, 12, ces derniers ayant une fonction de maintien de l'élément de capture 2.

De préférence, lesdits membres s'étendent sensiblement perpendiculairement au corps 10.

Le premier membre 11 comporte un orifice traversant 110, dont les dimensions sont adaptées pour permettre le passage de la portion 20' sans frottement de la surface annulaire de l'élément 2 portant l'échantillon prélevé, afin de ne pas endommager celui-ci.

Le second membre 12 est quant à lui adapté pour maintenir la portion 20' une fois qu'elle a été introduite à travers l'orifice 110.

Par exemple, le second membre 12 comporte un orifice 120, qui est de préférence borgne (c'est-à-dire non traversant) pour garantir le positionnement axial de l'élément 2 dans le dispositif 1 et pour éviter que la portion 20' ne puisse ressortir du dispositif 1 lors de sa manipulation.

Une fois introduite dans le dispositif 1, la portion 20' est maintenue à ses extrémités en s'appuyant sur une surface annulaire intérieure des orifices 110 et 120, tandis que sa partie centrale, qui porte notamment l'élément de capture 2, est préservée de tout contact avec le corps 10 et avec le tube dans lequel le dispositif 1 doit être inséré.

Les membres 11 et 12 remplissent ainsi une fonction d'espacement de l'échantillon vis-à-vis du dispositif lui-même et du récipient dans lequel il doit être conservé.

Le dispositif 1 préserve également l'élément de capture 2 de toute contrainte mécanique (torsion, flexion).

De préférence, on oriente la surface portant l'échantillon à l'opposé du corps 10, de sorte à la rendre visible.

Ceci permet, le cas échéant, d'observer l'échantillon par exemple au moyen d'un microscope ou d'un appareil binoculaire.

L'orifice 120 est avantageusement conçu pour éviter toute rotation de la portion 20' une fois qu'elle a été mise en place. Par exemple, il est comblé par un matériau mou, tel une silicone, ce qui permet le blocage en rotation de la portion 20' lorsque cette dernière pénètre dans ce matériau.

Selon un mode de réalisation, les orifices 110 et 120 sont coaxiaux et leur axe est parallèle au corps 10.

De manière avantageuse, le corps 10 peut présenter un méplat 100 parallèle audit axe.

Ledit méplat permet de poser le dispositif 1 sur une surface telle que celle d'une table ou d'un appareil de visualisation de manière stable et en conservant l'élément de capture 2 parallèle à ladite surface.

De préférence, le dispositif 1, c'est-à-dire le corps 10 et les membres 11, 12, est inscrit dans un cylindre, de sorte que l'élément de capture 2 est maintenu à l'intérieur dudit cylindre.

D'autre part, le diamètre de ce cylindre est de préférence aussi proche que possible du diamètre du tube dans lequel l'échantillon doit être conservé.

Cette disposition permet en effet de minimiser l'encombrement du stockage et, lorsqu'un liquide doit être introduit dans le tube pour la conservation de l'échantillon, de minimiser le volume de liquide nécessaire.

Ceci permet également d'utiliser des tubes standard tels que les tubes dits « low bind » commercialisés par la société Eppendorf.

Par ailleurs, le premier membre 10 présente, sur une face opposée au second membre de maintien 12, un élément de préhension 111 adapté à la préhension du dispositif 1 par un instrument tel qu'une pince.

L'élément de préhension peut prendre toute forme appropriée, tenant compte notamment de l'instrument qui est envisagé pour la manipulation du dispositif 1.

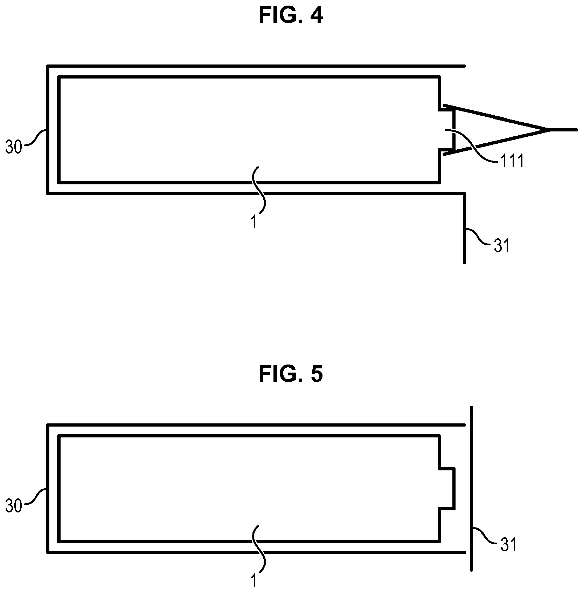

Par exemple, s'agissant d'une pince (cf. figure 4), l'élément de préhension 111 peut présenter avantageusement deux surfaces parallèles d'une superficie suffisante pour que les deux bras de la pince puissent y prendre appui.

Le dispositif 1 peut avantageusement être réalisé en un matériau polymère, présentant à la fois une faible adsorption des espèces biologiques (protéines, ADN, ARN) et une bonne résistance aux contraintes thermiques qu'implique la conservation de l'échantillon.

Par exemple, le dispositif 1 peut être en polyoxyméthylène (POM), en polyétheréthercétone (PEEK), en polypropylène (PP), ou en polyéthylène (PE) (liste non exhaustive).

Le dispositif 1 étant destiné à être jeté après chaque utilisation, il peut être commercialisé avec un dispositif de prélèvement tel que décrit plus haut, sous la forme d'un kit de prélèvement biologique.

Concrètement, une fois qu'un échantillon a été prélevé sur l'élément de prélèvement 2 porté par la tige 20, l'opérateur insère la portion distale 20' dans le dispositif 1, à travers l'orifice 110 jusqu'en butée au fond de l'orifice 120.

De préférence avant cette opération, le dispositif 1 a préalablement été inséré dans un tube 30 (cf. figure 5) destiné à sa conservation, le second membre 12 étant placé au fond du tube. Ceci évite toute contamination ou endommagement accidentel de l'échantillon lors de sa mise en place.

L'opérateur sectionne la portion distale 20', de préférence au moyen d'une zone sécable prévue sur la tige 20.

De manière avantageuse, on détache la portion 20' du reste de la tige 20 une fois que ladite portion 20' a été mise en place dans le dispositif 1, ce qui facilite son insertion à travers l'orifice 110.

Le cas échéant, l'opérateur remplit le tube d'un liquide approprié pour la conservation de l'échantillon.

Puis l'opérateur obture le tube au moyen d'un bouchon 31.

Le tube 30 peut ainsi être stocké dans les conditions requises jusqu'à l'analyse de l'échantillon.

Pour extraire le dispositif 1 du tube 30, l'opérateur utilise un instrument tel qu'une pince pour saisir l'élément de préhension 111 (cf. figure 4).

Les modes de réalisation décrits plus haut ne sont naturellement pas limitatifs et l'homme du métier pourra notamment faire varier les formes et proportions du dispositif sans pour autant sortir du cadre de la présente invention.

### REFERENCES

WO 2006/082344
WO 2008/006871

## Revendications

1. Dispositif (1) de conservation d'un échantillon biologique recueilli sur une surface d'un élément de capture (2) agencé sur une portion (20') détachable d'un dispositif de prélèvement biologique, comprenant :
- un corps (10),
- deux membres (11, 12) de maintien de ladite portion (20') solidaires dudit corps (10), lesdits membres de maintien (11, 12) étant agencés de sorte à maintenir entre eux ladite portion (20') en évitant tout contact entre la surface de l'élément de capture (2) et le corps (10),
- un premier membre (11) est pourvu d'un orifice (110) pour le passage sans frottement de l'élément de capture (2), le second membre de maintien étant pourvu d'un orifice borgne (120) adapté pour recevoir une extrémité de ladite portion (20').

2. Dispositif selon la revendication 1, **caractérisé en ce que** lesdits orifices (110, 120) sont coaxiaux et **en ce que** le corps (10) présente un méplat (100) parallèle à l'axe desdits orifices.

3. Dispositif selon l'une des revendications 1 à 2, **caractérisé en ce que** la distance entre lesdits membres de maintien (11, 12) est supérieure ou égale à la longueur de l'élément de capture (2) et inférieure à la longueur de la portion (20').

4. Dispositif selon l'une des revendications 1 à 3, **caractérisé en ce que** le premier membre de maintien (11) présente, sur une face opposée au second membre de maintien (12), un élément de préhension (111) adapté à la préhension du dispositif par un instrument.

5. Dispositif selon l'une des revendications 1 à 4, **caractérisé en ce qu'**il est inscrit dans un cylindre, l'élément de capture (2) étant maintenu à l'intérieur dudit cylindre.

6. Dispositif selon l'une des revendications 1 à 5, **caractérisé en ce qu'**il est réalisé en un matériau polymère.

7. Dispositif selon la revendication 6, **caractérisé en ce que** ledit matériau polymère est choisi parmi le polyoxyméthylène (POM), le polyétheréthercétone (PEEK), le polypropylène (PP) et le polyéthylène (PE).

8. Kit de prélèvement biologique, comprenant :
- un dispositif de prélèvement biologique comprenant une tige (20) et un élément (2) de capture de cibles biologiques disposé sur une portion distale (20') de la tige et détachable de ladite tige et
- au moins un dispositif (1) de conservation de l'échantillon biologique selon l'une des revendications 1 à 7.

9. Procédé de conservation d'un échantillon biologique prélevé au moyen d'un dispositif de prélèvement biologique comprenant une tige (20) et un élément (2) de capture de cibles biologiques disposé sur une portion distale (20') de la tige et détachable de ladite tige, **caractérisé en ce qu'**il comprend les étapes suivantes :
- fourniture d'un dispositif (1) de conservation selon l'une des revendications 1 à 7,
- introduction dudit dispositif (1) dans un tube (30) destiné à la conservation de l'échantillon,
- introduction de la portion (20') dans l'orifice (110) du premier membre de maintien et mise en place de ladite portion (20') entre les membres de maintien (11, 12),
- détachement de ladite portion (20') de la tige (20),
- fermeture du tube (30) par un bouchon (31).

10. Procédé selon la revendication 9, **caractérisé en ce qu'**il comprend l'introduction d'un liquide dans le tube (30).

11. Procédé selon l'une des revendications 9 ou 10, **caractérisé en ce que** le diamètre extérieur du dispositif (1) est sensiblement égal au diamètre intérieur du tube (30).

12. Procédé selon l'une des revendications 9 à 11, **caractérisé en ce que** la surface de l'élément de capture (2) est orientée à l'opposé du corps (10) de sorte à être visible à travers la paroi du tube (30).

## Patentansprüche

1. Vorrichtung (1) zur Aufbewahrung einer biologischen Probe, die auf einer Oberfläche eines Aufnahmeelements (2) gesammelt wurde, das auf einem abnehmbaren Abschnitt (20') einer Vorrichtung zur biologischen Probenentnahme angeordnet ist, umfassend:
- einen Körper (10),
- zwei Glieder (11, 12) zum Halten des Abschnitts (20'), die fest mit dem Körper (10) verbunden sind, wobei die Halteglieder (11, 12) so angeordnet sind, dass sie zwischen sich den Abschnitt (20') halten und dabei jeglichen Kontakt zwischen der Oberfläche des Aufnahmeelements (2) und dem Körper (10) vermeiden,
- ein erstes Glied (11), das mit einem Loch (110) für den reibungslosen Durchgang des Aufnahmeelements (2) ausgestattet ist, wobei das zweite Halteglied mit einem Sackloch (120) ausgestattet ist, das zum Aufnehmen eines Endes des Abschnitts (20') angepasst ist.

2. Vorrichtung nach Anspruch 1, **dadurch gekennzeichnet, dass** die Löcher (110, 120) koaxial sind und dadurch, dass der Körper (10) eine Abflachung (100) aufweist, die parallel zu der Achse der Löcher verläuft.

3. Vorrichtung nach einem der Ansprüche 1 bis 2, **dadurch gekennzeichnet, dass** der Abstand zwischen den Haltegliedern (11, 12) größer als oder gleich wie die Länge des Aufnahmeelements (2) und kleiner als die Länge des Abschnitts (20') ist.

4. Vorrichtung nach einem der Ansprüche 1 bis 3, **dadurch gekennzeichnet, dass** das erste Halteglied (11) auf einer Fläche, die dem zweiten Halteglied (12) gegenüberliegt, ein Greifelement (111) aufweist, das zum Ergreifen der Vorrichtung mittels eines Instruments angepasst ist.

5. Vorrichtung nach einem der Ansprüche 1 bis 4, **dadurch gekennzeichnet, dass** sie in einen Zylinder passt, wobei das Aufnahmeelement (2) im Inneren des Zylinders gehalten wird.

6. Vorrichtung nach einem der Ansprüche 1 bis 5, **dadurch gekennzeichnet, dass** sie aus einem Polymermaterial hergestellt ist.

7. Vorrichtung nach Anspruch 6, **dadurch gekennzeichnet, dass** das Polymermaterial gewählt wird aus Polyoxymethylen (POM), Polyetheretherketon (PEEK), Polypropylen (PP) und Polyethylen (PE).

8. Besteck zur biologischen Probenentnahme, umfassend:
- eine Vorrichtung zur biologischen Probenentnahme, die einen Stab (20) und ein Aufnahmeelement (2) für biologische Zielstrukturen umfasst, das auf einem distalen Abschnitt (20') des Stabs und abnehmbar von dem Stab eingerichtet ist, und
- mindestens eine Vorrichtung (1) zur Aufbewahrung der biologischen Probe nach einem der Ansprüche 1 bis 7.

9. Verfahren zur Aufbewahrung einer biologischen Probe, die mittels einer Vorrichtung zur biologischen Probenentnahme entnommen wurde, die einen Stab (20) und ein Aufnahmeelement (2) für biologische Zielstrukturen umfasst, das auf einem distalen Abschnitt (20') des Stabs und abnehmbar von dem Stab eingerichtet ist, **dadurch gekennzeichnet, dass** es folgende Schritte umfasst:
- Bereitstellen einer Vorrichtung (1) zur Aufbewahrung nach einem der Ansprüche 1 bis 7,
- Einführen der Vorrichtung (1) in eine Röhre (30), die zur Aufbewahrung der Probe bestimmt ist,
- Einführen des Abschnitts (20') in das Loch (110) des ersten Halteglieds und Einrichten des Abschnitts (20') zwischen den Haltegliedern (11, 12),
- Abnehmen des Abschnitts (20') von dem Stab (20),
- Verschließen der Röhre (30) mittels einer Kappe (31) .

10. Verfahren nach Anspruch 9, **dadurch gekennzeichnet, dass** es das Einführen einer Flüssigkeit in die Röhre (30) umfasst.

11. Verfahren nach einem der Ansprüche 9 oder 10, **dadurch gekennzeichnet, dass** der Außendurchmesser der Vorrichtung (1) im Wesentlichen gleich ist wie der Innendurchmesser der Röhre (30).

12. Verfahren nach einem der Ansprüche 9 bis 11, **dadurch gekennzeichnet, dass** die Oberfläche des Aufnahmeelements (2) dem Körper (10) gegenüberliegend ausgerichtet ist, so dass sie durch die Wand der Röhre (30) sichtbar ist.

## Claims

1. Device (1) for preserving a biological sample collected on a surface of a capturing element (2) arranged on a detachable portion (20') of a biological sampling device, comprising:
- a body (10),
- two holding members (11, 12) for holding said portion (20') integral with said body (10), said holding members (11, 12) being arranged so as to hold said portion between them (20') avoiding any contact between the surface of the capturing element (2) and the body (10),
- a first member (11) provided with an opening (110) for the passage without friction of the capturing element (2), the second holding member being provided with a blind opening (120) suitable for receiving an end of said portion (20').

2. Device according to claim 1, **characterised in that** said openings (110, 120) are coaxial and **in that** the body (10) has a flat part (100) parallel to the axis of said openings.

3. Device according to one of claims 1 to 2, **characterised in that** the distance between said holding members (11, 12) is greater than or equal to the length of the capturing element (2) and less than the length of the portion (20').

4. Device according to one of claims 1 to 3, **characterised in that** the first holding member (11) has, on a face opposite the second holding member (12), a gripping member (111) suitable for gripping the device by an instrument.

5. Device according to one of claims 1 to 4, **characterised in that** it is inscribed in a cylinder, with the capturing element (2) being held inside said cylinder.

6. Device according to one of claims 1 to 5, **characterised in that** it is made of a polymer material.

7. Device according to claim 6, **characterised in that** said polymer material is chosen from polyoxymethylene (POM), polyetherethercetone (PEEK), polypropylene (PP) and polyethylene (PE).

8. Biological sampling kit, comprising:
- a biological sampling device comprising a rod (20) and an element (2) for capturing biological samples arranged on a distal portion (20') of the rod and which can be detached from said rod and
- at least one device (1) for preserving the biological sample according to one of claims 1 to 7.

9. Method for preserving a biological sample sampled using a biological sampling device comprising a rod (20) and an element (2) for capturing biological targets arranged on a distal portion (20') of the rod and which can be detached from said rod, **characterised in that** it comprises the following steps:
- provision of a preserving device (1) according to one of claims 1 to 7,
- introduction of said device (1) into a tube (30) intended for preserving the sample,
- introduction of the portion (20') into the orifice (110) of the first holding member and setting in place of said portion (20') between the holding members (11, 12),
- detaching of said portion (20') from the rod (20),
- closing of the tube (30) by a plug (31).

10. Method according to claim 9, **characterised in that** it comprises the introduction of a liquid into the tube (30).

11. Method according to one of claims 9 or 10, **characterised in that** the outer diameter of the device (1) is substantially equal to the inner diameter of the tube (30).

12. Method according to one of claims 9 to 11, **characterised in that** the surface of the capturing element (2) is directed opposite the body (10) in such a way as to be visible through the wall of the tube (30) .
